# EUROPÄISCHE PATENTSCHRIFT

(11) **EP 3 129 348 B1**
(45) Veröffentlichungstag und Bekanntmachung des Hinweises auf die Patenterteilung: **02.05.2018**
(21) Anmeldenummer: 15711222.8
(22) Anmeldetag: 23.03.2015
(51) Int. Cl.: C07C 67/10, C07C 51/09, C07C 53/08, C07C 69/01, C07C 67/343, C07C 51/353

(54) **VERFAHREN ZUR RUTHENIUM-KATALYSIERTEN UMVINYLIERUNG VON CARBONSÄUREN**
PROCESS FOR RUTHENIUM-CATALYZED TRANSVINYLATION OF CARBOXYLIC ACIDS
PROCÉDÉ DE VINYLISATION D'ACIDES CARBOXYLIQUES CATALYSÉE PAR LE RUTHÉNIUM

(30) Priorität: 10.04.2014 DE 102014206916
(43) Veröffentlichungstag der Anmeldung: 15.02.2017
(73) Patentinhaber: Wacker Chemie AG, 81737 München (DE)
(72) Erfinder: GIGLER, Peter, 85221 Dachau (DE); LEUTE, Maria, 84489 Burghausen (DE); STOHRER, Jürgen, 82049 Pullach (DE)
(74) Vertreter: Schuderer, Michael
(86) Internationale Anmeldenummer: PCT/EP2015/056109
(87) Internationale Veröffentlichungsnummer: WO 2015/154979

(56) Entgegenhaltungen:
- EP-A2- 0 351 603
- EP-A2- 0 497 340

## Beschreibung

Die Erfindung betrifft ein Verfahren zur Umvinylierung einer Eduktcarbonsäure mit einem Eduktvinylester zu einem Produktvinylester und der korrespondierenden Säure des Eduktvinylesters in Gegenwart von einem oder mehreren Rutheniumkatalysatoren.

Die Umvinylierung von Carbonsäuren dient der Darstellung von Vinylestern. Darunter versteht man die Übertragung einer Vinyl-Einheit eines Eduktvinylesters (1V) auf eine Eduktcarbonsäure (2S) unter Generierung eines Produktvinylesters (2V) und der korrespondierenden Säure des Eduktvinylesters (1S).

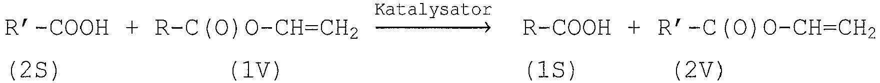

Aus der EP 376075 B1 ist die Umvinylierung von Vinylestern mit Carbonsäuren in Gegenwart von Palladiumkatalysator bekannt, wobei Kupferbromid und spezielle Lithiumverbindungen als Cokatalysatoren eingesetzt werden.

Neben Palladium-Katalysatoren und Quecksilber-Katalysatoren werden im Stand der Technik zur Umvinylierung von Vinylestern mit Carbonsäuren auch Ruthenium-Verbindungen als Katalysator eingesetzt. Ruthenium-Verbindungen zeichnen sich durch ihre hohe Löslichkeit, geringe Flüchtigkeit und hohe thermische Stabilität aus. Hinzu kommt eine hohe, Temperatur-induzierbare Aktivität.

In der EP 351603 A2 (= EP 506070, US 4981973, US 5155253) wird ein Verfahren zur Umvinylierung von Carbonsäuren unter Verwendung verschiedener Ru-Verbindungen als Katalysatoren beschrieben. Die Autoren postulieren eine [Ru(CO)₂RCO₂]-Einheit als entscheidendes Strukturelement bei der Bildung der aktiven Spezies. Demzufolge können alle Ru-Verbindungen als Katalysatoren eingesetzt werden, die sich in situ in dieses Strukturelement umwandeln lassen. Als geeignete Ausgangsspezies wird unter anderem der industriell verfügbare trinukleare Ru-Komplex [Ru₃O(OAc)₆(H₂O)₃]OAc genannt und es wird festgestellt, dass dieses carbonyl-freie Ruthenium-Carboxylat auch unter Stickstoff- statt Kohlenstoffmonoxid-Atmosphäre in die aktive Katalysatorspezies überführt wird. In Beispiel 2, 5, 6 und 14 wird dieser Komplex als Katalysator in der Umvinylierung verschiedener Carbonsäuren eingesetzt. Während die Umsetzung in den Beispielen 2, 5 und 6 unter Kohlenstoffmonoxid-Atmosphäre und bei Reaktionsdauern von 3 bzw. 4,5 Stunden stattfindet, erfolgt die Umvinylierung in Beispiel 14 bei 100°C unter Stickstoffatmosphäre. Die dabei signifikant längere Reaktionsdauer von 19 h ist auf eine verlangsamte Formierung der aktiven Katalysatorspezies in Abwesenheit von Kohlenstoffmonoxid zurückzuführen. Der Einsatz von [Ru₃O(OAc)₆(H₂O)₃]OAc in einem kontinuierlichen Verfahren wird nicht beschrieben.

In Adv. Synth. Catal. 2013, 355, 2845 - 2859 wird die Theorie der EP 351603 A2 bestätigt und ein [Ru(CO)₃(RCO₂)₂]-Komplex als aktive Katalysatorspezies postuliert. Die Bildung der katalytisch aktiven Spezies erfolgt in diesem Falle auf Basis von RuCl₃. Es werden Ru-Carbonyl-Propionat und Ru-Carbonyl-Valerat durch Umsetzung von RuCl₃ mit Propionsäure oder Valeriansäure hergestellt. Carbonylfreie Rutheniumcarboxylate werden nicht eingesetzt.

Die EP 497340 A2 (US5210207) beschreibt ein Umvinylierungsverfahren zur Darstellung von Produktvinylestern, deren Siedepunkte höher liegen als die der Eduktvinylester. Durch Reaktivdestillation von mindestens einer der Produktkomponenten wird das Gleichgewicht der Reaktion auf die Produktseite verschoben. Bevorzugt werden hierfür die in EP 351603 A2 beschriebenen Ru-Katalysatoren eingesetzt. In Beispiel 8 wird als Katalysator [Ru₃O(OAc)₆(H₂O)₃]OAc eingesetzt, wobei darauf hingewiesen wird, dass der Umsatz im Gegensatz zu einem Ru-Carbonyl-Carboxylat verlangsamt ist. Dies legt nahe, dass die Umsetzung des eingesetzten [Ru₃O(OAc)₆(H₂O)₃]OAc zur aktiven Katalysatorspezies unter den Reaktionsbedingungen von Beispiel 8 nur langsam erfolgt.

In der WO 92/09554 A1 wird ein Verfahren beschrieben, bei dem die Reaktionsmasse nach der Umvinylierung zuerst separiert und der Produktvinylester anschließend durch Azeotropdestillation abgetrennt wird. Dieses Verfahren zielt vor allem auf die Trennung von Säure/Vinylester-Gemischen mit geringen Siedepunktdifferenzen. In der Umvinylierungsreaktion werden bevorzugt Ru-Katalysatoren der EP 351603 A2 eingesetzt. Der Einsatz von carbonylfreien Rutheniumcarboxylaten als Katalysator wird in den Beispielen nicht beschrieben.

Die WO 2013/117294 A1 beschreibt ein kontinuierliches Verfahren zur Darstellung von Carbonsäurevinylestern. Die übergangsmetallkatalysierte Umvinylierung wird im stationären Zustand betrieben und das Reaktionsgemisch in einem Folgeschritt aufgetrennt. WO 2013/117295 beschreibt eine weitere Ausgestaltung dieses Verfahrens mit einer nachträglichen Derivatisierung der entstehenden, konjugaten Säure des Eduktvinylesters. In den Beispielen beider Schriften werden hauptsächlich Pd-Katalysatoren für die Umvinylierung eingesetzt. In zwei Beispielen wird als Ru-Katalysator Ruthenium-Dicarbonylacetat eingesetzt. Carbonylfreie Rutheniumcarboxylate werden als Katalysatoren nicht beschrieben.

Die Verwendung von Ru-Katalysatoren in der Umvinylierungsreaktion birgt deutliche Vorteile gegenüber Pd-Katalysatoren hinsichtlich Löslichkeit, Flüchtigkeit, thermische Stabilität und thermisch induzierbarer Aktivität. Zahlreiche Ru-Verbindungen lassen sich in situ zu aktiven Ru-Spezies umwandeln. Aufgrund ihrer großtechnischen Verfügbarkeit sind dabei vor allem Ruthenium(III)chlorid und der trinukleare, carbonylfreie Ru-Acetat-Komplex [Ru₃O(OAc)₆(H₂O)ₙ(AcOH)₃₋ₙ]OAc mit n = 0 bis 3, oder dessen (Lösungsmittel-freies) Analogon [Ru₃O(OAc)₆]OAc vorteilhaft. Während ersterer durch Zugabe einer Base erst formiert werden muss, benötigt der Ru-Acetat-Komplex gemäß dem Stand der Technik eine Kohlenstoffmonoxid-Atmosphäre um in kurzen Reaktionszeiten zur aktiven Spezies umgewandelt zu werden. Die Vermeidung einer Kohlenstoffmonoxid-Zuführung ist aus sicherheitstechnischen Gründen jedoch höchst erstrebenswert. Ein Verfahren, in dem ein kommerziell verfügbarer Katalysator ohne Zusatz von Kohlenstoffmonoxid innerhalb kurzer Reaktionszeiten in die aktive Spezies überführt wird und daher für den Einsatz in kontinuierlichen Umvinylierungsprozessen geeignet ist, ist bislang unbekannt.

Es bestand daher die Aufgabe ein Umvinylierungsverfahren zu entwickeln, bei dem der eingesetzte Katalysator ohne Zusatz von Kohlenstoffmonoxid innerhalb der Verweilzeit in der Reaktionszone in die katalytisch aktive Spezies überführt wird.

Gegenstand der Erfindung ist ein Verfahren zur Umvinylierung einer Eduktcarbonsäure mit einem Eduktvinylester zu einem Produktvinylester und der korrespondierenden Säure des Eduktvinylesters in Gegenwart von einem oder mehreren Rutheniumkatalysatoren, wobei
a) der Eduktvinylester, die Eduktcarbonsäure und ein Ruthenium-katalysator dem Reaktor zugeführt werden, und
b) die Umvinylierungsreaktion durchgeführt wird,
dadurch gekennzeichnet, dass
als Rutheniumkatalysator ein carbonylfreies Ru(III)-Carboxylat eingesetzt wird, und kein Kohlenstoffmonoxid zugeführt wird,
c) die Reaktion bei einer Temperatur von 110°C bis 170°C durchgeführt wird
d) nach Abschluss der Umvinylierungsreaktion der Eduktvinylester und die korrespondierende Säure aus dem Reaktionsgemisch destillativ abgetrennt werden,
e) aus dem Sumpfprodukt der Destillation der Produktvinylester destillativ abgetrennt wird, und
f) das verbleibende Reaktionsgemisch in den Reaktor zurückgeführt wird.

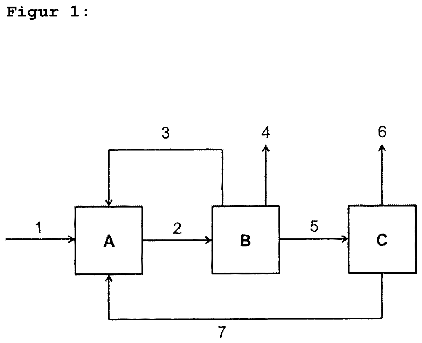

Der prinzipielle Ablauf des erfindungsgemäßen Verfahrens ist in Figur 1 dargestellt. Die Edukte (1) werden einem Reaktor (A) einzeln oder als Gemisch zugeführt. Im Reaktor (A) erfolgt die Umvinylierungsreaktion. Das resultierende Reaktionsgemisch (2) wird in einer Destillationsvorrichtung (B) von Eduktvinylester (3) und dessen korrespondierender Säure (4) befreit. Der Eduktvinylester (3) wird gegebenenfalls dem Reaktor (A) zurückgeführt. Vom verbleibenden Produktgemisch (5) wird anschließend der Produktvinylester (6) in einer Destillationsvorrichtung (C) vollständig oder teilweise abgetrennt. Der zurückbleibende Katalysatorhaltige Reaktionssumpf (7) wird in den Reaktor (A) zurückgeführt und der Katalysator somit erneut eingesetzt.

Als Reaktor (A) können Rührkessel, Rührkessel-Kaskaden oder Rohrreaktoren eingesetzt werden. Vorzugsweise ist der Reaktor (A) ein Rohrreaktor.

Als Eduktvinylester können beliebige Carbonsäurevinylester der allgemeinen Formel R-C(O)O-CH=CH₂ eingesetzt werden, wobei R ein aliphatischen Rest mit 1 bis 12 C-Atomen sein kann, oder ein cycloaliphatischer Rest mit bis zu 12 C-Atomen sein kann, oder ein aromatischer Rest mit bis zu 12 C-Atomen sein kann. Bevorzugt ist die Verwendung niedermolekularer Eduktvinylester, wobei R ein Alkylrest mit 1 bis 6 C-Atomen ist, beispielsweise Vinylacetat, Vinylpropionat und Vinylpivalat. Besonders bevorzugt ist die Verwendung von Vinylacetat.

Ferner wird dem Reaktor mindestens eine Eduktcarbonsäure der allgemeinen Formel R'-COOH zugeführt, wobei R' ein aliphatischer Rest mit 1 bis 22 C-Atomen sein kann, oder ein cycloaliphatischer Rest mit bis zu 22 C-Atomen sein kann, oder ein aromatischer Rest mit bis zu 22 C-Atomen sein kann. Bevorzugt werden Eduktcarbonsäuren der genannten Verbindungsklassen mit 6 bis 18 C-Atomen eingesetzt. Beispiele hierfür sind Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure, Laurinsäure, Myristinsäure, Palmitinsäure, Stearinsäure, Benzoesäure, Naphthalincarbonsäure. Besonders bevorzugt werden Versaticsäuren^{R} (alphaverzweigte Carbonsäuren mit 9 bis 12 C-Atomen der Fa. Momentive) oder Neo-Säuren mit 9 bis 12 C-Atomen und Fettsäuren wie Laurinsäure, Myristinsäure, Palmitinsäure, Stearinsäure.

Als Katalysator wird ein carbonylfreies Ru(III)-Carboxylat eingesetzt. Die Zugabe kann in fester und gelöster Form erfolgen. Bevorzugt wird das carbonylfreie Ru(III)-Carboxylat in gelöster Form eingesetzt. Als Carboxylate können Carboxylate von Carbonsäuren der allgemeinen Formel R"-COOH eingesetzt werden, wobei R" ein aliphatischer Rest mit 1 bis 22 C-Atomen sein kann, oder ein cycloaliphatischer Rest mit bis zu 22 C-Atomen sein kann, oder ein aromatischer Rest mit bis zu 22 C-Atomen sein kann. Beispiele hierfür sind die Carboxylate folgender Carbonsäuren: Essigsäure, Propionsäure, n-Buttersäure, iso-Buttersäure, n-Valeriansäure, 2-Methylbuttersäure, 3-Methylbüttersäure, Pivalinsäure, Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure, Laurinsäure, Myristinsäure, Palmitinsäure, Stearinsäure, Benzoesäure, Naphthalincarbonsäure. Bevorzugt ist die Verwendung eines Ru(III)-Acetats.

Die carbonylfreien Ru(III)-Carboxylate sind kommerziell erhältlich, beispielsweise das Ru(III)-Carboxylat der Formel [Ru₃O(OAc)₆(H₂O)ₙ(AcOH)₃₋ₙ]OAc mit n =0 bis 3 oder der Formel [Ru₃O(OAc)₆]OAc, wobei Ac jeweils für einen Rest CH₃-C=O steht. Die genannten carbonylfreien Ru(III)-Carboxylate sind auch durch Umsetzung von RuCl₃ mit der entsprechenden Carbonsäure mittels dem Fachmann bekannter Verfahren zugänglich. [Ru₃O(OAc)₆(H₂O)ₙ(AcOH)₃₋ₙ]OAc mit n = 0 bis 3 oder [Ru₃O(OAc)₆]OAc kann beispielsweise mittels Umsetzung von RuCl₃ mit Essigsäure und Natriumacetat in Ethanol erhalten werden.

Als Lösungsmittel eignen sich die gerade beschriebenen Carbonsäuren. Bevorzugt ist die Verwendung von Essigsäure. Die Konzentration des carbonylfreien Ru(III)-Carboxylats im Lösungsmittel kann 0,01 bis 50 Gew.-% betragen, bevorzugt 0,1 bis 20 Gew.-%, besonders bevorzugt 1 bis 10 Gew.-%.

Besonders bevorzugt wird eine Lösung von Ruthenium(III)-Acetat in Essigsäure, am meisten bevorzugt eine Lösung von [Ru₃O(OAc)₆(H₂O)ₙ(AcOH)₃₋ₙ]OAc mit n =0 bis 3 oder [Ru₃O(OAc)₆]OAc jeweils in Essigsäure eingesetzt.

Der Rutheniumkatalysator wird typischerweise in Konzentrationen von 0,1 bis 10000 ppm (Gehalt Ruthenium bezogen auf die Reaktionsmasse aus Eduktvinylester und Eduktcarbonsäure) eingesetzt, bevorzugt ist die Verwendung von 1 bis 1000 ppm (Gehalt Ruthenium bezogen auf die Reaktionsmasse aus Eduktvinylester und Eduktcarbonsäure).

Den Edukten kann gegebenenfalls ein Polymerisationsinhibitor zugesetzt werden. Bevorzugt werden 100 bis 10000 ppm, bezogen auf die Reaktionsmasse aus Eduktvinylester und Eduktcarbonsäure, Polymerisationsinhibitor eingesetzt. Beispiele für Polymerisationsinhibitoren sind Hydrochinon, Methoxyhydrochinon, tertiär-Butylcatechol, Phenothiazin oder Nitroxidradikale wie TEMPO oder 4-OH-TEMPO (TEMPO = 2,2,6,6-Tetramethylpiperidinyloxyl). Bevorzugt ist die Verwendung von Phenothiazin oder Hydrochinon.

Gegebenenfalls kann auch ein Anhydrid der jeweiligen Eduktcarbonsäure als Edukt zugegeben werden. Die gegebenenfalls zugeführten Anhydride der Eduktcarbonsäure der allgemeinen Formel R¹-C(O)-O-C(O)-R² können als gemischte (R¹ ≠ R²) oder symmetrische (R¹ = R²) Anhydride vorliegen, wobei R¹ und R² jeweils einen aliphatischen Rest mit 1 bis 22 C-Atomen, oder einen cycloliphatischen Rest mit bis zu 22 C-Atomen oder einen aromatischen Rest mit bis zu 22 C-Atomen darstellt. Beispiele hierfür sind gemischte oder symmetrische Anhydride folgender Säuren: Essigsäure, Propionsäure, n-Buttersäure, iso-Buttersäure, n-Valeriansäure, 2-Methylbuttersäure, 3-Methylbuttersäure, Pivalinsäure, Capronsäure, Cyclohexancarbonsäure, n-Heptansäure, 2-Methylhexansäure, 2 Ethylhexansäure, n-Octansäure, n-Nonansäure, Isononansäure, Neononansäure, n-Decansäure, Neodecansäure, Laurinsäure, Myristinsäure, Palmitinsäure, Stearinsäure, Benzoesäure, Naphtalincarbonsäure. Bevorzugt werden die symmetrischen Anhydride der Eduktcarbonsäure eingesetzt.

Zur Umvinylierung können die Reaktanden Eduktvinylester, Eduktcarbonsäure und Ru-Katalysator, sowie gegebenenfalls Inhibitor sowie gegebenenfalls Anhydrid der Eduktcarbonsäure, dem Reaktor einzeln oder in einem Gemisch zugeführt werden.

Das molare Verhältnis von Eduktvinylester zu Eduktcarbonsäure kann 1 : 10 bis 10 : 1 betragen. Bevorzugt ist ein Verhältnis von Eduktvinylester zu Eduktcarbonsäure von 1 : 2 bis 8 : 1, besonders bevorzugt ist ein Verhältnis von 1 : 1 bis 6 : 1.

Die Umvinylierung wird im Allgemeinen bei einer Temperatur von 110°C bis 170°C, vorzugsweise bei einer Temperatur von 120°C bis 150°C durchgeführt. Der Druck, bei welchem die Umvinylierung erfolgt, ist von der Temperatur abhängig und beträgt im Allgemeinen ≥ 2 bar abs., vorzugsweise 5 bis 15 bar abs., und am meisten bevorzugt 5 bis 10 bar abs.. Die Reaktion wird ohne Zuführung von Kohlenstoffmonoxid und bevorzugt in einer Schutzgasatmosphäre, beispielsweise Stickstoff, in an sich bekannter Weise, durchgeführt.

Die Verweilzeit im Reaktor beträgt bei dem erfindungsgemäßen Verfahren im Allgemeinen 0,25 bis 5 Stunden, vorzugsweise 1 Stunde bis 4 Stunden.

Im Unterschied zu einer Reaktivdestillation erfolgt im erfindungsgemäßen Verfahren die Auftrennung des erhaltenen Produktgemisches erst nach Abschluss der Umvinylierung vorzugsweise mittels Destillation in entsprechenden Destillationskolonnen. Druck und Temperatur der Destillation sowie die Auslegung der Destillationskolonnen hängen von den im Produktgemisch vorliegenden Komponenten ab und können beispielsweise mittels Routineversuchen vom Fachmann ermittelt werden. Bei dem erfindungsgemäßen Verfahren wird folglich keine Reaktivdestillation oder Azeotropdestillation durchgeführt.

Bei der Auftrennung des Produktgemisches werden in einem ersten Schritt der nicht umgesetzte Rest des Eduktvinylesters und dessen korrespondierende Säure jeweils aus dem Produktgemisch abgetrennt. Der damit erhaltene Eduktvinylester kann gegebenenfalls zur neuerlichen Umvinylierung in den Reaktor zurückgeführt werden. Die damit erhaltene korrespondierende Säure des Eduktvinylesters kann als Edukt in anderen chemischen Prozessen eingesetzt werden; im Falle von Essigsäure beispielsweise zur Herstellung von Vinylacetat.

In einer bevorzugten Ausführungsform wird der Produktvinylester aus dem, nach Abtrennung des Eduktvinylesters und dessen korrespondierende Säure, verbleibenden Produktgemisch durch Destillation zumindest teilweise oder vollständig abgetrennt. Der resultierende Reaktionssumpf, welcher Eduktcarbonsäure, Anhydride der Eduktcarbonsäure, Ruthenium-Katalysator und gegebenenfalls weitere Komponenten wie Produktvinylester oder polymere Bestandteile enthalten kann, wird unter Zusatz von frischen Edukten und gegebenenfalls frischem, carbonylfreiem Ru(III)-Carboxylat in den Reaktor für eine neuerliche Umvinylierung zurückgeführt.

Der Zusatz von frischen Edukten und gegebenenfalls frischem, carbonylfreiem Ru(III)-Carboxylat in den Reaktor für eine neuerliche Umvinylierung kann jeweils im Gemisch mit dem zurückgeführten Reaktionssumpf erfolgen oder jeweils getrennt vom zurückgeführten Reaktionssumpf erfolgen.

Die Teilschritte des Verfahrens, sowohl die Umvinylierung als auch die Aufarbeitungsschritte, können diskontinuierlich, semikontinuierlich und vollkontinuierlich durchgeführt werden. Das Verfahren wird bevorzugt vollkontinuierlich durchgeführt.

Mit dem erfindungsgemäßen Verfahren können carbonylfreie Ru(III)-Carboxylat-Komplexe als Katalysatoren in der Umvinylierung von Carbonsäuren eingesetzt werden. Überraschend wurde gefunden, dass bei Temperaturen von ≥ 110°C, selbst bei Verweilzeiten in der Reaktionszone von kleiner als 5 h die Ru-Carboxylat-Spezies in die aktive Spezies überführt werden kann.

Durch das erfindungsgemäße Verfahren ist keine Kohlenstoffmonoxid-Zuführung nötig. Ferner wird der Einsatz kommerziell erhältlicher Katalysatoren in kontinuierlichen Umvinylierungsprozessen möglich.

### Beispiele:

Die nachfolgenden Beispiele dienen der näheren Erläuterung der Erfindung.

Die angegebenen Umsätze beziehen sich in allen Fällen auf die im geringeren molaren Anteil eingesetzte Ausgangskomponente Eduktcarbonsäure (2S) oder Eduktvinylester (IV). Der Umsatz U ist definiert als U (%) = 100 x (n₀-n_{E})/n₀, wobei n₀ die Stoffmenge der Ausgangskomponente zu Beginn der Reaktion und n_{E} die Stoffmenge am Ende der Reaktion darstellt.

### Vergleichsbeispiel 1:

Umvinylierung mit Ru-Acetat-Lösung als Katalysator bei 100°C.

In einem 100 ml Berghoff-Autoklav wurden 25,0 g (125 mmol) Laurinsäure, 43,0 g (500 mmol) Vinylacetat und 0,69 g (0,9 mmol) [Ru₃O(OAc)₆(H₂O)₃]OAc (4,5 Gew.-% Ru, gelöst in Essigsäure der Fa. Umicore) bei 2,0 bar abs. 6 Stunden lang auf 100°C erhitzt.

### Beispiel 2:

Umvinylierung mit Ru-Acetat-Lösung als Katalysator bei 140°C.

In einem 100 ml Berghoff-Autoklav wurden 25,0 g (125 mmol) Laurinsäure, 43,0 g (500 mmol) Vinylacetat und 0,69 g (0,9 mmol) [Ru₃O(OAc)₆(H₂O)₃]OAc (4,5 Gew.-% Ru, gelöst in Essigsäure der Fa. Umicore) bei 6,0 bar abs. 5 Stunden lang auf 140°C erhitzt.

In beiden Fällen wurden in definierten Zeitabständen Proben genommen und die für die Berechnung von Umsatz benötigten molaren Anteile im Reaktionsgemisch mittels quantitativer NMR-Spektroskopie ermittelt.

| Zeit [h] | Umsatz Laurinsäure[%] | |
|---|---|---|
| | V.Bsp. 1 100°C | Bsp. 2 140°C |
| 0 | 0 | 0 |
| 1,5 | 0, 9 | 78,4 |
| 3 | 1,6 | 79,7 |
| 4,5 | 2,6 | 79,3 |

Das Beispiel zeigt, dass bei Verwendung von Ruthenium-Acetat-Lösung als Katalysator bei einer Temperatur von 140°C die Bildung der katalytisch aktiven Spezies innerhalb Verweilzeiten kleiner 5 Stunden erfolgt. Bereits nach 1,5 Stunden ist der Gleichgewichtszustand der Reaktion erreicht.

## Patentansprüche

1. Verfahren zur Umvinylierung einer Eduktcarbonsäure mit einem Eduktvinylester zu einem Produktvinylester und der korrespondierenden Säure des Eduktvinylesters in Gegenwart von einem oder mehreren Rutheniumkatalysatoren, wobei
a) der Eduktvinylester, die Eduktcarbonsäure und ein Rutheniumkatalysator dem Reaktor zugeführt werden, und
b) die Umvinylierungsreaktion durchgeführt wird,
**dadurch gekennzeichnet, dass**
als Rutheniumkatalysator ein carbonylfreies Ru(III)-Carboxylat eingesetzt wird, und kein Kohlenstoffmonoxid zugeführt wird,
c) die Reaktion bei einer Temperatur von 110°C bis 170°C durchgeführt wird
d) nach Abschluss der Umvinylierungsreaktion der Eduktvinylester und die korrespondierende Säure aus dem Reaktionsgemisch destillativ abgetrennt werden,
e) aus dem Sumpfprodukt der Destillation der Produktvinylester destillativ abgetrennt wird, und
f) das verbleibende Reaktionsgemisch in den Reaktor zurückgeführt wird.

2. Verfahren nach Anspruch 1, **dadurch gekennzeichnet, dass** die Verweilzeit in der Reaktionszone 0,25 bis 5 Stunden beträgt.

3. Verfahren nach Anspruch 1 oder 2, **dadurch gekennzeichnet, dass** die Reaktion bei einer Temperatur von 120°C bis 150°C durchgeführt wird.

4. Verfahren nach Anspruch 1 bis 3, **dadurch gekennzeichnet, dass** [Ru₃O(OAc)₆(H₂O)ₙ(AcOH)₃₋ₙ]OAc mit n = 0 bis 3 oder [Ru₃O(OAc)₆]OAc als Rutheniumkatalysator eingesetzt wird.

5. Verfahren nach Anspruch 1 bis 4, **dadurch gekennzeichnet, dass** als Rutheniumkatalysator eine Lösung von [Ru₃O(OAc)₆(H₂O)ₙ(AcOH)₃₋ₙ]OAc mit n = 0 bis 3 oder [Ru₃O(OAc)₆]OAc jeweils in Essigsäure eingesetzt wird.

6. Verfahren nach Anspruch 1 bis 5, **dadurch gekennzeichnet, dass** als Eduktvinylester ein Carbonsäurevinylester der allgemeinen Formel R-C(O)O-CH=CH₂ eingesetzt wird, wobei R ein aliphatischen Rest mit 1 bis 12 C-Atomen ist, oder ein cycloaliphatischer Rest mit bis zu 12 C-Atomen ist, oder ein aromatischer Rest mit bis zu 12 C-Atomen ist.

7. Verfahren nach Anspruch 1 bis 5, **dadurch gekennzeichnet, dass** als Eduktvinylester Vinylacetat eingesetzt wird.

8. Verfahren nach Anspruch 1 bis 7, **dadurch gekennzeichnet, dass** als Eduktcarbonsäure eine Carbonsäure der allgemeinen Formel R'-COOH eingesetzt wird, wobei R' ein aliphatischer Rest mit 1 bis 22 C-Atomen ist, oder ein cycloaliphatischer Rest mit bis zu 22 C-Atomen ist, oder ein aromatischer Rest mit bis zu 22 C-Atomen ist.

9. Verfahren nach Anspruch 1 bis 7, **dadurch gekennzeichnet, dass** als Eduktcarbonsäure eine Carbonsäure eingesetzt wird aus der Gruppe umfassend Versaticsäuren und Neo-Säuren, jeweils mit 9 bis 12 C-Atomen, und Fettsäuren wie Laurinsäure, Myristinsäure, Palmitinsäure, Stearinsäure.

10. Verfahren nach Anspruch 1 bis 7, **dadurch gekennzeichnet, dass** ein Anhydrid der jeweiligen Eduktcarbonsäure als Edukt zugegeben wird.

## Claims

1. Process for transvinylation of a reactant carboxylic acid with a reactant vinyl ester to afford a product vinyl ester and the corresponding acid of the reactant vinyl ester in the presence of one or more ruthenium catalysts, wherein
a) the reactant vinyl ester, the reactant carboxylic acid and a ruthenium catalyst are supplied to the reactor, and
b) the transvinylation reaction is performed,
**characterized in that**
the ruthenium catalyst employed is a carbonyl-free Ru(III) carboxylate and no carbon monoxide is supplied,
c) the reaction is performed at a temperature of 110°C to 170°C,
d) after completion of the transvinylation reaction the reactant vinyl ester and the corresponding acid are distillatively removed from the reaction mixture, and
e) the product vinyl ester is distillatively removed from the bottoms product of the distillation, and
f) the remaining reaction mixture is recycled into the reactor.

2. Process according to Claim 1, **characterized in that** the residence time in the reaction zone is 0.25 to 5 hours.

3. Process according to Claim 1 or 2, **characterized in that** the reaction is performed at a temperature of 120°C to 150°C.

4. Process according to Claim 1 to 3, **characterized in that** the ruthenium catalyst employed is [Ru₃O(OAc)₆(H₂O)ₙ(AcOH)₃₋ₙ]OAc where n = 0 to 3 or [Ru₃O(OAc)₆]OAc.

5. Process according to Claim 1 to 4, **characterized in that** the ruthenium catalyst employed is a solution, in each case in acetic acid, of [Ru₃O(OAc)₆(H₂O)ₙ(AcOH)₃₋ₙ]OAc where n =0 to 3 or Ru₃O(OAc)₆]OAc.

6. Process according to Claim 1 to 5, **characterized in that** the reactant vinyl ester employed is a vinyl carboxylate ester of general formula R-C(O)O-CH=CH₂, wherein R is an aliphatic radical having 1 to 12 carbon atoms or is a cycloaliphatic radical having up to 12 carbon atoms or is an aromatic radical having up to 12 carbon atoms.

7. Process according to Claim 1 to 5, **characterized in that** the reactant vinyl ester employed is vinyl acetate.

8. Process according to Claim 1 to 7, **characterized in that** the reactant carboxylic acid employed is a carboxylic acid of general formula R'-COOH, wherein R' is an aliphatic radical having 1 to 22 carbon atoms or is a cycloaliphatic radical having up to 22 carbon atoms or is an aromatic radical having up to 22 carbon atoms.

9. Process according to Claim 1 to 7, **characterized in that** the reactant carboxylic acid employed is a carboxylic acid selected from the group comprising Versatic acids and neo acids, each having 9 to 12 carbon atoms, and fatty acids such as lauric acid, myristic acid, palmitic acid, stearic acid.

10. Process according to Claim 1 to 7, **characterized in that** an anhydride of the respective reactant carboxylic acid is added as a reactant.

## Revendications

1. Procédé de transvinylation d'un acide carboxylique réactif avec un ester de vinyle réactif pour former un ester de vinyle produit et l'acide correspondant de l'ester de vinyle réactif en présence d'un ou de plusieurs catalyseurs de ruthénium, selon lequel
a) l'ester de vinyle réactif, l'acide carboxylique réactif et un catalyseur de ruthénium sont introduits dans le réacteur, et
b) la réaction de transvinylation est réalisée,
**caractérisé en ce que**
un carboxylate de Ru(III) sans carbonyle est utilisé en tant que catalyseur de ruthénium et aucun monoxyde de carbone n'est introduit,
c) la réaction est réalisée à une température de 110 °C à 170 °C,
d) après la fin de la réaction de transvinylation, l'ester de vinyle réactif et l'acide correspondant sont éliminés par distillation du mélange réactionnel,
e) l'ester de vinyle produit est séparé par distillation du produit de fond, et
f) le mélange réactionnel restant est recyclé dans le réacteur.

2. Procédé selon la revendication 1, **caractérisé en ce que** le temps de séjour dans la zone de réaction est de 0,25 à 5 heures.

3. Procédé selon la revendication 1 ou 2, **caractérisé en ce que** la réaction est réalisée à une température de 120 °C à 150 °C.

4. Procédé selon les revendications 1 à 3, **caractérisé en ce que** [Ru₃O(OAc)₆(H₂O)ₙ(AcOH)₃₋ₙ] OAc avec n = 0 à 3 ou [Ru₃O(OAc)₆]OAc est utilisé en tant que catalyseur de ruthénium.

5. Procédé selon les revendications 1 à 4, **caractérisé en ce qu'**une solution de [Ru₃O(OAc)₆(H₂O)ₙ(AcOH)₃₋ₙ]OAc avec n = 0 à 3 ou de [Ru₃O(OAc)₆]OAc à chaque fois dans de l'acide acétique est utilisée en tant que catalyseur de ruthénium.

6. Procédé selon les revendications 1 à 5, **caractérisé en ce qu'**un ester de vinyle d'acide carboxylique de formule générale R-C(O)O-CH=CH₂ est utilisé en tant qu'ester de vinyle réactif, R étant un radical aliphatique contenant 1 à 12 atomes C, ou un radical cycloaliphatique contenant jusqu'à 12 atomes C, ou un radical aromatique contenant jusqu'à 12 atomes C.

7. Procédé selon les revendications 1 à 5, **caractérisé en ce que** de l'acétate de vinyle est utilisé en tant qu'ester de vinyle réactif.

8. Procédé selon les revendications 1 à 7, **caractérisé en ce qu'**un acide carboxylique de formule générale R'-COOH est utilisé en tant qu'acide carboxylique réactif, R' étant un radical aliphatique contenant 1 à 22 atomes C, ou un radical cycloaliphatique contenant jusqu'à 22 atomes C, ou un radical aromatique contenant jusqu'à 22 atomes C.

9. Procédé selon les revendications 1 à 7, **caractérisé en ce qu'**un acide carboxylique du groupe comprenant les acides versatiques et les néo-acides, à chaque fois contenant 9 à 12 atomes C, et les acides gras tels que l'acide laurique, l'acide myristique, l'acide palmitique, l'acide stéarique, est utilisé en tant qu'acide carboxylique réactif.

10. Procédé selon les revendications 1 à 7, **caractérisé en ce qu'**un anhydride de l'acide carboxylique réactif respectif est ajouté en tant que réactif.
